**Europäisches Patentamt**

**European Patent Office**

**Office européen des brevets**

(11) Numéro de publication : **0 362 049 B1**

(12) **FASCICULE DE BREVET EUROPEEN**

(19)

(45) Date de publication du fascicule du brevet :
**13.05.92 Bulletin 92/20**

(21) Numéro de dépôt : **89402633.5**

(22) Date de dépôt : **26.09.89**

(51) Int. Cl.⁵ : **F16B 2/18, A61F 5/02**

(54) **Système d'assemblage de deux organes rigides plans et utilisation pour l'assemblage d'éléments d'armature d'un corsetorthopédique.**

(30) Priorité : **28.09.88 FR 8812677**

(43) Date de publication de la demande :
**04.04.90 Bulletin 90/14**

(45) Mention de la délivrance du brevet :
**13.05.92 Bulletin 92/20**

(84) Etats contractants désignés :
**DE ES IT**

(56) Documents cités :
**AT-B- 13 385**
**DE-A- 3 522 533**
**FR-A- 2 199 363**

(73) Titulaire : **ETABLISSEMENTS PROTEOR**
**11 rue des Buttes**
**F-21100 Dijon (FR)**

(72) Inventeur : **Vera, Bernard**
**30 Rue de la République**
**F-21250 Seurre (FR)**

(74) Mandataire : **Jolly, Jean-Pierre et al**
**Cabinet Jolly 54, rue de Clichy**
**F-75009 Paris (FR)**

EP 0 362 049 B1

Jouve, 18, rue Saint-Denis, 75001 PARIS

## Description

La présente invention concerne un système d'assemblage de façon amovible de deux organes rigides sensiblement plans. L'invention concerne également l'utilisation de ce système pour l'assemblage d'éléments d'armature d'un corset orthopédique.

C'est à cette utilisation que l'on se référera essentiellement dans la suite de la présente description, mais le système d'assemblage que l'on définira ci-après n'est pas limite, naturellement, à cette application.

On sait que les corsets orthopédiques comprennent des pièces moulées en matière thermoplastique, qui, maintenues en appui sous pression contre des parties du thorax à l'aide d'une armature rigide, généralement métallique, permettent d'exercer une action de correction de déviation des os du patient. Cette armature rigide comprend habituellement un ou des tuteurs disposés verticalement, sur lesquels sont fixés latéralement des éléments rigides de liaison et/ou des charnières, supportant les pièces en matière plastique.

Les diverses pièces métalliques sont généralement percées de rangées de trous filetés intérieurement ou de fentes allongées, qui permettent de les assembler à l'aide de vis en une pluralité de positions (voir AT-B-13 385). Le corset peut ainsi être appliqué sur le corps en exerçant une pression sur les organes à maintenir ou à redresser. En outre, il est possible d'assembler les différentes pièces en des positions diverses, ce qui permet d'exercer avec le corset des actions de correction progressives.

Il est cependant nécessaire de retirer fréquemment le corset du corps du patient, par exemple pour faire effectuer à ce dernier des exercices respiratoires ou pour lui appliquer un traitement de kinésithérapie, le corset devant ensuite être remis en place. Le système usuel d'assemblage par vis se révèle alors très peu pratique, car les différentes vis doivent être dévissées une à une des pièces assemblées, lors du démontage du corset, puis remises en place à la fin du traitement, ce qui est long et fastidieux.

La présente invention vise à remédier à ces inconvénients en proposant un système d'assemblage des éléments d'armature du corset, qui utilise toujours les trous taraudes et les vis de la technique antérieure, ce qui permet d'assembler les éléments en différentes positions et d'exercer ainsi avec le corset une correction progressive des déformations osseuses, mais qui se prête aussi à une solidarisation ou à une désolidarisation simples et rapides de ces éléments, lorsque l'on désire retirer le corset du corps du patient ou le remettre en place.

A cet effet, l'invention a pour objet un système d'assemblage de façon amovible de deux organes rigides, le premier organe comportant une surface plane percée d'une rangée de trous taraudés aptes à recevoir une vis à tête, tandis que le second organe présente une partie destinée à venir au contact de la surface du premier organe percée des trous, cette partie étant elle-même percée d'une fente longitudinale d'une largeur sensiblement égale au diamètre de trous, ce système étant caractérisé en ce que:

– une portion de ladite fente est suffisamment large pour laisser passer librement la tête de ladite vis;

– la partie du second élément destinée à venir au contact du premier élément est en un matériau rigide se déformant élastiquement et présente une partie galbée dont la concavité est tournée vers la surface contiguë du premier élément ;

– un élément est monté libre en rotation autour de la tige de ladite vis, entre la tête de celle-ci et la surface du premier organe percée de trous taraudes, et a une dimension transversale telle qu'il puisse passer librement dans la portion de la fente du second organe apte à laisser passer la tête de la vis ;

– un moyen de commande en rotation de cet élément comporte deux parties latérales, aptes à être amenées par rotation, d'une part, dans une première position où elles sont en contact avec les surfaces convexes de la partie galbée du second organe bordant la fente, de manière à solliciter ces surfaces en direction de la surface percée de trous du premier organe, d'autre part, dans une seconde position, où elles sont dirigées selon la direction générale de la fente; le moyen de commande en rotation ayant des dimensions telles que, dans cette seconde position, il puisse passer librement à travers cette fente;

– et, de façon connue en soi, deux vis engagées dans deux trous du premier organe de part et d'autre de la vis à tête, de manière que leurs tiges fassent saillie à l'intérieur de ladite fente, pour former des butées d'alignement de la fente sur la rangée de trous taraudés.

La présente invention a également pour objet l'utilisation d'un tel système d'assemblage pour réunir de façon amovible des éléments de l'armature d'un corset orthopédique.

Comme on l'exposera plus en détail ci-après, dans la position où le moyen de commande en rotation de l'élément monté libre en rotation par rapport à la vis à tête sollicite la partie galbée du second organe en direction du premier organe, cette partie agit comme un ressort et les deux organes sont assemblés dans cette position. Dans le cas où ces organes sont des parties de l'armature d'un corset orthopédique, ils se trouvent alors dans la position où le corset est fermé, en position d'utilisation, et enserre le thorax du patient. Pour ouvrir le corset et en débarasser le patient, il suffit donc de faire pivoter le moyen de commande en rotation pour l'amener dans la position où il ne sollicite plus le second organe, mais où il peut passer librement à travers la fente de celui-ci pour désolidariser le second organe du premier. En effet, dans cette position, aussi bien la tête de la vis solidaire du premier organe que l'élément interposé entre cet organe et la tête de la vis peuvent aussi passer à travers la fente du deuxième organe. Pour séparer le second organe du premier organe, il suffira donc d'imprimer un simple mouvement de rotation de 90° au moyen de commande en rotation, comme on le verra ci-après, sans qu'il soit nécessaire de dévisser la vis solidaire du premier élément.

Par contre, à mesure que progresse la rééducation d'un patient et qu'il devient nécessaire de modifier les positions relatives des différentes parties de son corset, il est toujours possible, comme dans les corsets de la technique connue, de retirer la vis à tête du trou taraudé qu'elle occupe sur le premier organe pour la visser dans un autre trou taraudé, l'assemblage du premier et du second organes rigides grâce au système conforme à l'invention se faisant alors en utilisant la vis dans sa nouvelle position.

L'élément monté libre en rotation pourra être une simple bague enfilée sur la tige de la vis à tête. Quant au moyen de commande en rotation de cet élément, il pourra s'agir d'un simple étrier solidaire de cet élément et disposé suivant un plan axial de la vis à tête, deux branches de cet étrier opposées diamétralement constituant les parties qui sollicitent le second organe rigide. Cet étrier sera de préférence monté pivotant autour de l'axe constitué par lesdites branches, de manière à pouvoir être amené dans un plan axial de la vis à tête pour être actionné en rotation, et pour pouvoir ensuite être rabattu contre le second organe, de manière à être escamoté, par exemple dans le position d'utilisation du corset, pour ne pas gêner le patient.

Pour les organes rigides, on s'est référé jusqu'à présent à des organes métalliques, mais il est bien évident que l'on peut utiliser n'importe quel matériau suffisamment rigide et présentant une élasticité suffisante pour pouvoir agir à la manière d'un ressort en position galbée. On pourra ainsi utiliser diverses matières plastiques et, plus particulièrement, des matières thermoplastiques armées de fibres de carbone.

L'invention sera décrite ci-après plus en détail en référence aux dessins annexés, qui, bien entendu, n'ont pas de caractère limitatif. Sur ces dessins :

La figure 1 est une vue en perspective de deux éléments rigides et du système d'assemblage conforme à l'invention, en position désolidarisée ;

La figure 2 est une vue analogue à la figure 1, en position assemblée des deux éléments rigides ;

La figure 3 est une coupe suivant la flèche F de la figure 2.

Les éléments rigides 1 et 2 représentés sur les dessins sont destinés à être assemblés au moyen du système conforme à l'invention.

L'élément 1 est un tuteur longitudinal plan, percé de deux rangées parallèles de trous taraudés identiques 3, destinés à recevoir des vis à tête telles que 4. Ce tuteur 1 peut constituer, par exemple, un élément vertical d'un corset orthopédique, destiné à supporter en des positions réglables des éléments de matière plastique appliqués sous pression contre le thorax d'un patient.

L'élément 2 est, dans le cas présent, une barrette coudée, présentant deux branches perpendiculaires 5 et 6.

Dans la branche 5, destinée à être rendue solidaire de façon amovible du tuteur 1, est pratiquée une fente longitudinale 7 de largeur sensiblement égale au diamètre des trous taraudés 3. Une partie de la fente 7 s'élargit en 7a, de manière à présenter une dimension suffisante pour permettre le libre passage de la tête de la vis 4.

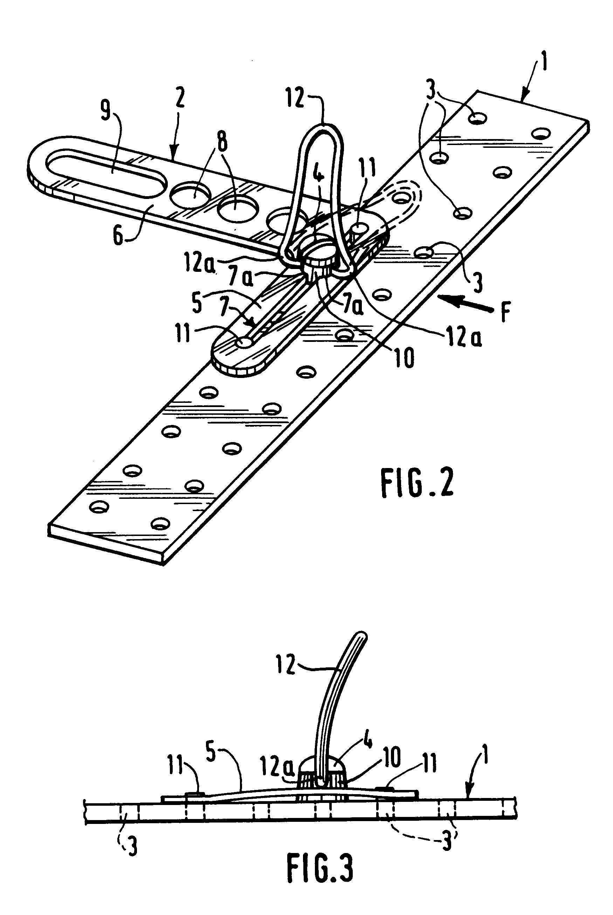

Cette branche 5 est galbée longitudinalement (voir figure 3), sa face concave étant tournée vers l'élément 1 et destinée à prendre appui contre celui-ci.

Dans la branche 6 sont pratiqués des trous 8 et une fente longitudinale 9 destinés à permettre la fixation sur cette branche à l'aide de vis ou de rivets des éléments en matière plastique moulée ou coquilles constituant le corset proprement dit.

Entre la tête de la vis 4 et la face contiguë de l'élément 1 est interposée une bague 10, enfilée sur la tige de la vis 4 et montée libre en rotation par rapport à celle-ci, ce qui suppose que la tête de la vis ne serre pas cette bague contre l'élément 1. La bague 10 a un diamètre externe inférieur ou égal à celui de la tête de la vis, de manière à pouvoir, elle aussi, traverser librement la partie 7a de la fente 7.

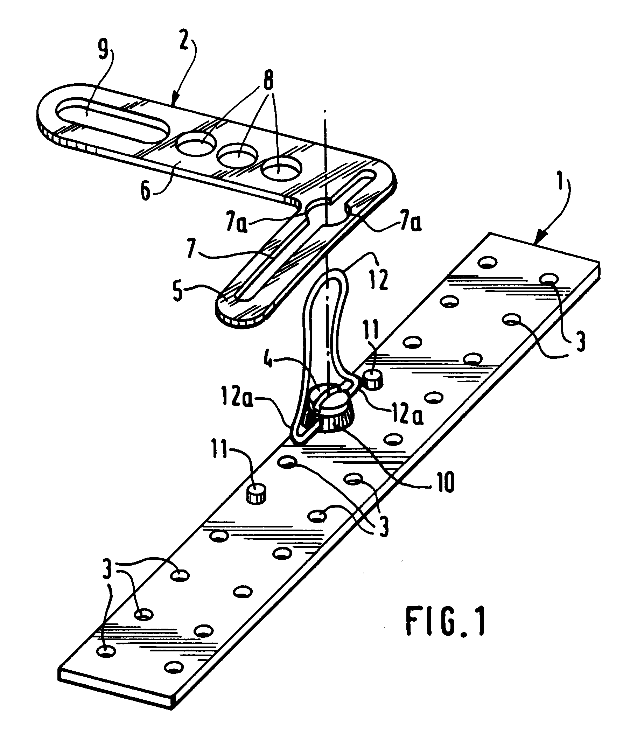

Des tétons 11 sont vissés dans des trous taraudés 3 de la même rangée que le trou dans lequel est vissée la vis 4, de part et d'autre de celle-ci, ces tétons 11 faisant saillie à la surface de l'élément 1 contre laquelle est destinée à prendre appui la branche 5 de l'élément 2. Comme on le voit sur la figure 1, les parties en saillie des tétons 11 sont destinées à être engagées dans la fente 7, aux deux extrémités de celle-ci, pour former des butées d'alignement de cette fente sur la rangée de trous 3 et de guidage en position de la branche 5.

Un étrier 12, monté pivotant par rapport à la bague 10 suivant un plan diamétral de celle-ci, permet de l'entraîner en rotation par rapport à la vis 4. Les deux branches inférieures 12a de cet étrier, sensiblement parallèles à la face contiguë de l'élément 1, prennent appui, en position d'assemblage des éléments 1 et 2, contre la face convexe de la branche 5 non tournée vers l'élément 1 et sollicitent cette face sous pression en direction de l'élément 1. L'élément 2 est en un matériau rigide apte à se déformer élastiquement, sous la pression de l'étrier 12, et il peut aussi être appliqué fermement par déformation contre l'élément 1 pour être pincé entre celui-ci et les branches 12a de l'étrier 12, et être rendu solidaire de l'élément 1. Avantageusement, des méplats peuvent être ménagés à la base des branches 12a de l'étrier 11, pour aider au maintien de celui-ci en position verrouillée.

Pour désolidariser les éléments 1 et 2, il suffit de faire pivoter l'étrier 12 de 90° à partir de sa position de la figure 1 pour amener ses branches 12a dans la direction de la fente 7. Elles ne sont plus alors en contact avec la branche 5 de l'élément 2 et celui-ci n'est donc plus pincé contre l'élément 1. L'étrier 12, la bague 10 dont il est solidaire et la vis 4 peuvent alors être dégagées de la fente 7 en passant à travers la partie élargie 7a de cette fente.

Les opérations de solidarisation et de désolidarisation des deux éléments sont donc d'une très grande simplicité et se prêtent particulièrement bien à la mise en place d'un corset orthopédique sur le corps d'un patient et à son retrait momentané de celui-ci.

On notera que, lorsque les éléments d'armature sont assemblés, l'étrier 12 peut être rabattu contre la branche 5 de l'élément 2, afin d'être escamoté et de ne gêner en rien l'utilisateur du corset.

## Revendications

1. Système d'assemblage de façon amovible de deux organes rigides, le premier organe (1) comportant une surface plane percée d'une rangée de trous taraudés (3) aptes à recevoir une vis à tête (4), tandis que le second organe (2) présente une partie (5) destinée à venir au contact de la surface du premier organe (1) percée de trous, cette partie (5) étant elle-même percée d'une fente longitudinale (7) d'une largeur sensiblement égale au diamètre des trous (3), caractérisé en ce que :
   – une portion (7a) de la fente longitudinale (7) est suffisamment large pour laisser passer librement la tête de la vis (4) ;
   – la partie (5) du second élément (2) destinée à venir au contact du premier élément (1) est en un matériau rigide se déformant élastiquement et présente une partie galbée dont la concavité est tournée vers la surface contiguë du premier élément ;
   – un élément (10) est monté libre en rotation autour de la tige de ladite vis (4), entre la tête de celle-ci et la surface du premier organe (1) percée de trous taraudés, et a une dimension transversale telle qu'il puisse passer librement dans la portion (7a) de la fente (7) du second organe (2) apte à laisser passer la tête de la vis (4) ;
   – un moyen (12) de commande en rotation de cet élément comporte deux parties latérales (12a), aptes à être amenées par rotation, d'une part, dans une première position en contact avec les surfaces convexes de la partie galbée (5) du second organe bordant la fente (7), de manière à solliciter ces surfaces en direction de la surface percée de trous du premier organe (1), d'autre part, dans une seconde position, où elles sont dirigées selon la direction générale de la fente (7), le moyen (12) de commande en rotation ayant des dimensions telles que, dans cette seconde position, il puisse passer librement à travers cette fente ;
   – et, de façon connue en soi, deux tétons (11) vissés dans deux trous (3) du premier organe de part et d'autre de la vis à tête (4), de manière à faire saillie à l'intérieur de ladite fente (7), pour former des butées d'alignement de la fente sur la rangée de trous taraudés.

2. Système selon la revendication 1, caractérisé en ce que l'élément monté libre en rotation par rapport à la vis à tête (4) est une bague (10) de diamètre inférieur ou égal à celui de la tête de la vis (4).

3. Système selon l'une des revendications 1 et 2, caractérisée en ce que le moyen d'entraînement en rotation de l'élément monté libre en rotation est un étrier (12), dont deux branches inférieures (12a) sensiblement parallèles à la surface de l'élément (1) percée de trous sont aptes à prendre appui sur la face convexe de la partie galbée de la partie (5) de l'élément (2).

4. Système selon la revendication 3, caractérisé en ce que l'étrier (12) est monté pivotant par rapport à l'élément (10) monté libre en rotation, autour d'un axe disposé perpendiculairement à l'axe de la vis à tête (4), de manière à pouvoir être escamoté par pivotement.

5. Utilisation d'une système selon l'une des revendications 1 à 4 pour l'assemblage de façon amovible des éléments de l'armature d'un corset orthopédique.

## Patentansprüche

1. System zum lösbaren Verbinden zweier starrer Elemente, wovon das erste (1) eine ebene Fläche aufweist, die zur Aufnahme einer Kopfschraube (4) von einer Reihe Gewindebohrungen (3) durchzogen ist, während das zweite Element (2) einen Abschnitt (5) aufweist, der mit der gelochten Fläche des ersten Elements (1) in Berührung bringbar ist, wobei dieser Abschnitt (5) seinerseits von einem Längsschlitz (7) durchzogen ist, dessen Breite im wesentlichen gleich dem Durchmesser der Löcher (3) ist, dadurch gekennzeichnet,
   – daß ein Abschnitt (7a) des Längsschlitzes (7) breit genug für den ungehinderten Durchtritt des Kopfes der Schraube (4) ist;
   – daß der Abschnitt (5) des zur Kontaktierung des ersten Elements vorgesehenen zweiten Elements (2) aus einem steifen Material besteht, das sich elastisch verformt und einen gewolbten Abschnitt aufweist, dessen Hohlfläche zur angrenzenden Fläche des ersten Elements zugekehrt ist;

– daß ein Teil (10) frei um den Schaft der Schraube (4) drehbar zwischen deren Kopf und der Fläche des ersten mit Gewindebohrungen versehenen Elements (1) angeordnet und in Querrichtung so bemessen ist, daß es ungehindert durch den Abschnitt (7a) des Schlitzes (7) im zweiten Element (2), durch welchen der Kopf der Schraube (4) hindurchführbar ist, führbar ist;

– daß eine Steuereinrichtung (12) zum Ansteuern der Drehbewegung dieses Teils zwei seitliche Abschnitte (12a) aufweist, welche einerseits durch Drehung in eine erste Stellung bringbar sind, in welcher sie die konvexen Flächen des gewölbten Abschnitts (5) des an den Schlitz (7) angrenzenden zweiten Elements so berühren, daß sie diese Flächen in Richtung der gelochten Fläche des ersten Elements (1) spannen, und andererseits in eine zweite Stellung, in welcher sie in der allgemeinen Richtung des Schlitzes (7) ausgerichtet sind, wobei die Steuereinrichtung (12) zum Ansteuern der Drehbewegung so bemessen ist, daß sie in der zweiten Stellung ungehindert durch diesen Schlitz führbar ist;

– und daß in an sich bekannter Weise zwei Ansätze (11) in zwei Löcher (3) des ersten Elements beiderseits der Kopfschraube (4) so eingeschraubt sind, daß sie in den Innenraum des Schlitzes (7) so vorstehen, daß sie Anschläge zur Ausrichtung des Schlitzes zur Reihe Gewindebohrungen bilden.

2. System nach Anspruch 1, dadurch gekennzeichnet, daß das gegenüber der Kopfschraube (4) drenbewegliche Teil ein Ring (10) ist, dessen Durchmesser kleiner als der Durchmesser oder gleich dem Durchmesser des Kopfes der Schraube (4) ist.

3. System nach einem der Ansprüche 1 und 2, dadurch gekennzeichnet, daß die Steuereinrichtung zum Ansteuern der Drehbewegung des drehbeweglich angeordneten Teils ein Bügel (12) ist, durch dessen beide untere Schenkel (12a), die im wesentlichen parallel zur Fläche des Elements (1) verlaufen, Löcher eingelassen sind, während die beiden unteren Schenkel auf der konvexen Fläche des gewolbten Abschnitts des Abschnitts (5) des Elements (2) abstützbar sind.

4. System nach Anspruch 3, dadurch gekennzeichnet, daß der Bügel (12 gegenüber dem drehbeweglich angeordneten Teil (10) um eine Achse verschwenkbar angebracht ist, die senkrecht zur Achse der Kopfschraube (4) so verläuft, daß der Bügel durch Verschwenken einziehbar ist.

5. Verwendung eines Systems nach einem der Ansprüche 1 bis 4, zum lösbaren Zusammenbau von Stützelementen eines orthopädischen Korsetts.

## Claims

1. A system for the detachable assembly of two rigid members, the first member (1) comprising a flat surface perforated with a row of tapped holes (3) suitable for receiving a cap screw (4), while the second member (2) has a part (5) designed to come into contact with the surface of the first member (1) perforated with holes, this part (5) itself being perforated with a longitudinal slit (7) of a width substantially equal to the diameter of the holes (3), characterized in that:

– a portion (7a) of the longitudinal slit (7) is sufficiently wide to allow the cap of the screw (4) to pass freely therethrough;

– the part (5) of the second member (2) designed to come into contact with the first member (1) is of a rigid material which deform elastically and has a curved part the concavity of which is turned towards the contiguous surface of the first member ;

– an element (10) is mounted to rotate freely about the shank of said screw (4), between the cap thereof and the surface of the first member (1) perforated with tapped holes, and has a transverse dimension such that it may pass freely into the portion (7a) of the slit (7) of the second member (2) suitable for allowing passage of the cap of the screw (4) ;

– a means (12) of controlling the rotation of this element comprises two lateral parts (12a) capable of being brought by rotation, on the one hand, into a first position in contact with the convex surfaces of the curved part (5) of the second member bordering the slit (7), in such a way as to stress these surfaces in the direction of the perforated surface of the first member (1) and, on the other hand, into a second position where they are directed according to the general direction of the slit (7), the means (12) of rotation control having dimensions such that, in this second position, it may pass freely through this slit ;

– and, in a manner known per se, two studs (11) screwed into two holes (3) in the first member on each side of the cap screw (4), in such a way as to project inside said slit (7) to form abutments aligning the slit with the row of tapped holes.

2. A system according to claim 1, characterized in that the element mounted freely for rotation with respect to the cap screw (4) is a ring (10) of diameter smaller than or equal to the cap of the screw (4).

3. A system according to either one of claims 1 and 2, characterized in that the means of driving in rotation the element mounted freely for rotation is a stirrup (12), whose lower two branches (12a) substantially parallel with the surface of the member (1) perforated with holes are capable of resting on the convex face of the curved part of the part (5) of the member (2).

4. A system according to claim 3, characterized in that the stirrup (12) is mounted pivotally, with respect to the element (10) mounted freely for rotation, about an axis disposed perpendicularly to the axis of the cap screw (4), in such a way as to be able to be folded back by pivoting.

5. Use of a system according to any one of claims 1 to 4 for the detachable assembly of the stay elements of an orthopaedic corset.

FIG.1

FIG.2

FIG.3